# EUROPEAN PATENT SPECIFICATION

(11) **EP 0 579 703 B1**
(45) Date of publication and mention of the grant of the patent: **10.04.1996**
(21) Application number: 92908881.3
(22) Date of filing: 11.02.1992
(51) Int. Cl.: A61K 9/127

(54) **INHIBITION OF AGGREGATION OF DRUG CONTAINING PARTICLES**
HEMMUNG DER AGGREGATON ARZNEISTOFFHALTIGER PARTIKELN
PROCEDE D'INHIBITION DE L'AGREGATION DE PARTICULES CONTENANT UN MEDICAMENT

(43) Date of publication of application: 26.01.1994
(73) Proprietor: NeXstar Pharmaceuticals, Inc., Boulder, CO 80301 (US)
(72) Inventor: ELEY, Crispin, G., S., Fullerton, CA 92635 (US); FORSSEN, Eric, A., LaCanada, CA 91011 (US)
(74) Representative: Brown, David Leslie
(86) International application number: US9201083
(87) International publication number: WO9315720

(56) References cited:
- WO-A-89/04656
- DAVID GLICK 'methods of biochemical analysis, volume 33 (liposomes: preparation, characterization, and preservation)' 1988 , INTERSCIENCE PUBLICATION JOHN WILEY

## Description

### Field of the Invention

This invention relates to the field of biochemistry and medicine, and more particularly to a method of preventing aggregation and fusion in pharmaceutical preparations which include particle-associated multivalent anionic moieties.

### Background and Summary of the Invention

Liposomes, also known as phospholipid vesicles, are known to be physiologically compatible particles which provide biodegradable delivery systems for a broad range of drugs. For example, U.S. Patent 5,019,369 teaches a method of targeting diagnostic and chemotherapeutic agents to tumors in the body of a patient by the intravenous administration of the agent encapsulated in unilamellar liposomes having a diameter of less than 200 nm.

Liposomes are microscopic delivery vesicles which are comprised of phospholipids which are polar molecules having a hydrophilic (ionizable) headgroup, and a hydrophobic tail consisting of fatty acid chains. Phospholipids form closed, fluid filled spheres when properly mixed with water. The hydrophobic tails spontaneously associate and exclude the water, while the hydrophilic phosphate ester headgroups are preferentially positioned toward the water.

The result is a spherical bilayer membrane in which the fatty acid tails point towards the interior of the membrane, and the polar heads point toward the aqueous medium. The polar heads at the inner surface of the membrane point toward the liposome's aqueous interior and those at the other (outer) surface point toward the exterior aqueous medium (*i.e*., the external continuous phase of the liposome dispersion). Liposomes may be either multilamellar, like an onion, with liquid separating many lipid bilayers, or unilamellar, with a single bilayer surrounding a liquid center. Finely divided phospholipids dispersed in aqueous solution spontaneously form bilayers, and simple agitation of the mixture usually produces multilamellar vesicles (MLVs), structures having diameters of 1-10 µm (1000-10,000 nm). Sonication of these structures, or other methods known in the art, leads to formation of unilamellar vesicles (UVs) having an average diameter of about 30-200 nm. The actual equilibrium diameter is largely determined by the nature of the phospholipid used, the suspending buffer, and the extent of incorporation of other lipids such as cholesterol. Standard methods for the formation of liposomes are known in the art, for example, methods for the commercial production of liposomes are described in U.S. Patent No. 4,753,788 to Ronald C. Gamble and Patent No. 4,935,171 to Kevin R. Bracken.

Liposomes for use in the invention can be prepared by any of the techniques now known in the art or subsequently developed. For example, the liposomes can be formed by the conventional technique for preparing MLVs, that is, by depositing a selected lipid on the inside wall of a suitable vessel by dissolving the lipid in chloroform, and then evaporating the dissolvent to leave a thin film on the inside of the vessel. An aqueous solution is then added to the vessel with a swirling or vortexing motion which results in the formation of large multilamellar vesicles.

Unilamellar vesicles can be prepared by reverse phase evaporation, infusion procedures, detergent dilution and sonication, that is, by providing a shear force necessary to form the smaller, unilamellar vesicles.

In addition to liposomes, other lipid particles such as those described in U.S. Patent 4,963,363 or other particles may be maintained for extended periods in a non-aggregative form, when the described anions are present on the outer surface of the particle according to the process of this invention.

It is also known to load liposomes with cationic, lipophilic drugs by first forming the liposomes in an aqueous medium in the presence of an organic acid which has multiple ionizable functional groups, and exchanging the unentrapped solution for a more basic (*i.e*., a solution having a higher pH) solution and adding a drug to the dispersion to load the drug into the liposomes. See, for example, U.S. Patent 4,946,683 to Forssen.

However, there may be potential problems with certain liposome dispersions, particularly those containing smaller liposomes or liposomes which include a multivalent anion disposed on the surface, in that vesicle aggregation or flocculation may occur upon prolonged storage in liquid form.

Accordingly, it has been a desideratum to provide a facile way to assure that aggregation of liposomes does not occur, thus extending the shelf life and optimizing the production of liposomes.

The present invention provides a method for the prevention of aggregation of lipid particles (*e.g*., liposomes) which are dispersed in an external aqueous medium (*i.e*., a continuous phase) and which include a multivalent anion, the method comprising the inclusion of a divalent cation in the continuous phase. Preferably, the divalent cation is selected from the group consisting of calcium and magnesium. In terms of the relative amounts of cation and lipids which are present in the dispersion, the divalent cation is present in an amount of up to 1.5:1 mole ratio with respect to total lipid content, preferably 0.002:1 to 0.9:1, most preferably 0.002:1 to 0.6:1. For most formulations, the divalent cation may be present in the continuous phase of the liposome dispersions in an amount of up to 50 mM, preferably 0.1 to 25 mM and most preferably from 0.1 to 16 mM to provide a reliable working range. While in the examples which follow the cation is added to the liposome dispersion, in other formulations the cation might be added to the hydrating buffer solution prior to liposome formation and have a similar anti-aggregative effect if the metal ion remains present in the external phase.

The divalent cation is added to the dispersion in the form of a salt, most preferably a salt selected from the group consisting chloride, bromide, iodide, nitrate, nitrite or carbonate. Most preferably, the divalent cation is calcium and is in the form of a soluble salt.

The multivalent anion of the liposome is selected from the group consisting of citrate, succinate, tartrate, oxalate, isocitrate, glutarate, fumarate, maleate, malonate, adipate, phthalate and dextran sulfate. The multivalent anion is usually found on the liposome in residual form, *e.g*., adhering to the liposome following a buffer replacement. The residual multivalent anion is thought to be a principal cause of aggregation in such liposomes.

### Detailed Description of the Invention

Biological lipids from which liposomal bilayer membrane particles or vesicles useful in practicing this invention can be prepared are amphiphilic (containing both a hydrophobic and hydrophilic portion) molecules which can spontaneously aggregate to form small spheres, ellipsoids or long cylinders, or bilayers having two or more parallel layers of amphiphilic molecules. In an aqueous (polar) medium, the polar heads of the amphiphilic molecules making up one layer orient outwardly to extend into the surrounding medium while the non-polar tail portions of these molecules likewise associate with each other. This provides a polar surface and a non-polar core in the wall of the vesicle. Such bilayered micelles usually take the shape of unilamellar (having one bilayer) or multilamellar (having a plurality of substantially concentric bilayers) spherical vesicles having an internal aqueous compartment.

Liposome bilayer membrane particles which have been found to be suitable in practicing this invention are small unilamellar vesicles having a mean diameter of less than 200 nm, preferably of from 30 to 150 nanometers (nm), and preferably from about 45 to about 60 nm, which are neutral (uncharged or having balanced charges; *i.e*., zwitterions) to induce specificity and tissue/cell targeting, thereby maximizing uptake of the resulting liposome drug delivery system.

Such liposome bilayer membrane particles include ones made from dipalmitoylphosphatidylcholine, distearoylphosphatidylcholine, dioleoylphosphatidylethanolamine, distearoylphosphatidylserine, dilinoleoylphosphatidylinositol, distearoylphosphatidylglycerol, and the like, or mixtures thereof. Liposome bilayer membrane particles made entirely from neutral phospholipids, such as distearoylphosphatidylcholine, and preferably ones which have been further stabilized with cholesterol or like-acting substances, for example in a molar ratio of distearoylphosphatidylcholine:cholesterol of about 2:1, respectively, have been found to be particularly suitable with regard to targeting efficiency when used to deliver anthracycline antineoplastic agents.

The invention is especially useful in preventing aggregation of particles containing cationic, ionizable lipophilic drugs, *e.g*., dibucaine, pilocarpine, quinine, prodipine, timolol, pentamidine, benadryl, dopamine, epinephrine, codeine, morphine, atropine, imipramine, quinidine, chloropromazine, and specifically anthracycline compounds having antineoplastic activity against cancerous tissues or cells, including daunorubicin, doxorubicin, mitoxantrone, aclacinomycin A, vinblastine, vincristine and mitomycin C.

While the particular type of particle, type and ratios of the included lipids, type of encapsulated drug and the various process parameters may affect the behavior and effect of the particle in several ways, for example, the ability of the particle to properly encapsulate bioactive agents, the operative factors which enable the practice of this invention are the presence of the described residual multivalent anions on the external surface of the particles, and the inclusion of the described divalent cation in the external phase of the particle dispersion.

In the examples which follow, liposomes are first formed by hydrating a dried lipid film or powder in an aqueous solution which includes a multivalent anion, preferably an organic acid anion. The acidity of the anion buffer solution may be acid or close to neutral pH, depending on the process employed for loading the drug.

The dispersion of lipids in the hydrating buffer is then homogenized in a modified Gaulin homogenizer (according to the method described in U.S. Patent 4,753,788) until liposomes of the appropriate size are formed. In general, the liposomes are heated during the homogenization step to a temperature that exceeds the transition temperature of the lipids which are employed. The drug is then added, under conditions which are specific to the particular method, and loaded into the liposomes. The divalent cation may be introduced at this step.

Preferably, the liposome dispersion is then ultrafiltered and washed with a buffer that contains the divalent cation. The liposomes are then sterile filtered.

Example 1 provides a description of liposomes which are loaded with daunorubicin by an ionic loading procedure. This procedure is described in the patent application PCT/US91/04807, filed July 3, 1991.

### Example 1

A mixture of chemically pure distearoyl phosphatidylcholine and cholesterol was dissolved in a 2:1 molar ratio in a chloroform/methanol solution. This solution was then dried to form a finely divided powder. The lipid powder was then hydrated in a buffer comprising 125 mM sucrose, 50 mM citric acid and 125 mM ethylenediamine at a pH of 7.5, at 65° to 72°C for one hour. This dispersion was then homogenized in the modified Gaulin homogenizer (according to the method described in U.S. Patent 4,753,788) at 689.5 bar (10,000 psi.) This procedure formed small unilamellar vesicles, which were then filtered through a 0.8 µm AAWP Millipore filter membrane at 65°C.

The liposome dispersion was then ultrafiltered in a Millipore Minitan tangential flow ultrafilter (with 100,000 nominal molecular weight limit [NMWL] polysulfone filters) with a wash buffer containing 250 mM sucrose and 50 mM glycine, to remove the external (unentrapped) citric acid and ethylenediamine.

The ultrafiltered liposomes were loaded by first heating the empty vesicles to 65°C. Daunorubicin hydrochloride was then added to the dispersion at a 17.4 to 1 lipid to drug ratio. The pH of the continuous phase was then adjusted to 7.5 with various combinations of base and/or calcium salt (as listed in the Table) to maintain a pH equal to that of the internal phase, and the dispersion was incubated at 65°C for 15 minutes. The dispersion was then allowed to cool to room temperature and the stability to aggregation was determined.

### Example 2

The procedure of Example 1 was followed in which the pH of the continuous phase was adjusted either by the addition of a combination of sodium hydroxide and calcium chloride to yield a final continuous phase calcium chloride concentration of 2 mM, or by sodium hydroxide alone. The liposomal drug dispersion was then ultrafiltered with the addition of calcium ions, according to the method of the invention, for the calcium chloride containing sample. First, the dispersion of daunorubicin containing liposomes was diluted 1:3 with a wash buffer which included 250 mM sucrose, 50 mM glycine and 2 mM calcium chloride. The dispersion was then ultrafiltered, in the 100,000 NMWL Minitan for a period of time sufficient to provide a one volume wash with the sucrose/glycine/cation wash buffer. The buffer influx was then temporarily stopped to concentrate the dispersion to 2.5 mg daunorubicin per ml. The ultrafiltration was then continued with the wash buffer until the conductivity of the eluate was reduced to the base line of the original wash buffer, *i.e*., about 360 micro MHO. The liposome dispersion was then sterile filtered to provide an aseptic therapeutic liposomal daunorubicin formulation. The liposomal sample pH adjusted without calcium chloride present, was similarly processed using the same wash buffer without calcium chloride. Sample stability to aggregation was then determined and results are shown in the Table.

### Example 3

Liposomes were loaded with daunorubicin according to the method described in U.S. Patent 4,946,683, the disclosure of which is incorporated herein by reference. A dried lipid powder comprising 2:1 DSPC:cholesterol was added to a hydrating buffer comprising 125 mM sucrose and a 50 mM citric acid (buffer pH 2.2) at 50°C and held for 3 minutes. This dispersion of lipids was then homogenized in a modified Gaulin homogenizer, described in U.S. Patent 4,946,683, at 703.29 bar (10.2 K psi) to produce unilamellar vesicles having a diameter of from 40 to 90 nm. Daunorubicin was loaded into these vesicles by first heating the vesicles in the hydrating buffer to 55°C, adding 3 grams per liter daunorubicin hydrochloride, adjusting the pH of the external (continuous) phase to 6.28 with either sodium hydroxide or sodium bicarbonate, and incubating the dispersion at 55°C for 20 minutes.

This dispersion of drug-loaded liposomes was then 0.8 µm filtered at 55°C and the pH was adjusted with 2.5 M HCl to pH 5.3.

The liposome dispersion was ultrafiltered, with the addition of a divalent cation according to the method of the invention. First, the dispersion of daunorubicin containing liposomes was diluted 1:3 with a wash buffer which included 250 mM sucrose, 50 mM glycine and one of several salts set forth below in the Table. The salts were added in varying amounts up to 20 mM. The dispersion was then ultrafiltered, in the 100,000 NMWL Minitan for a period of time sufficient to provide a one volume wash with the sucrose/glycine/cation wash buffer. The make up buffer flow was then temporarily stopped to concentrate the dispersion to 2.5 mg daunorubicin per ml. The ultrafiltration was then continued with the wash buffer until the conductivity of the eluate was reduced to the base line of the original wash buffer, *i.e.*, about 360 micro MHO. The liposome dispersion was then filtered through a 0.2 µm membrane to provide an aseptic therapeutic liposomal daunorubicin formulation.

The advantages of the invention are shown by the Table. The presence of the divalent cations calcium and magnesium, in amounts of up to 40 mM (0.74 to 1 mole ratio cation to lipid) inhibits the aggregation of the liposomes. As used herein, aggregation in a liposome dispersion is determined by the presence or formation of visible particulates in the dispersion or by an increase in solution turbidity. Experiments have also been performed with lipid concentrations which were 50% of several of those in the above Examples, *i.e.,* at cation to lipid ratios two fold higher than those in the Table. Similar results were shown with respect to the inhibition of aggregation.

From the foregoing description the essential characteristics of the invention can be readily ascertained in the context of the following claims.

**TABLE**

| Ex. | Final concentration of Cation in the External Phase of the Liposome Dispersion | | Aggregation | | |
|---|---|---|---|---|---|
| | mM | mole ratio cation/lipid | day 1 | day 7 | day 115 |
| 1 | none | 0 | yes | - | - |
| 1 | 1 mM CaCO₃ | 0.019:1 | none | - | - |
| 1 | 5 mM CaCO₃ | 0.093:1 | none | - | - |
| 1 | 10 mM CaCO₃ | 0.185:1 | none | - | - |
| 1 | 40 mM CaCO₃ | 0.741:1 | none | - | - |
| 1 | 1.25 mM NaHCO₃ | 0.023:1 | yes | - | - |
| 1 | 2.5 mM NaHCO₃ | 0.046:1 | yes | - | - |
| 1 | 5 mM NaHCO₃ | 0.093:1 | yes | - | - |
| 1 | 6.5 mM NaHCO₃ | 0.120:1 | yes | - | - |
| 1 | 12.5 mM NaHCO₃ | 0.231:1 | yes | - | - |
| 1 | 0 mM CaCl₂** | 0 | slight* | - | - |
| 1 | 1 mM CaCl₂** | 0.019:1 | none | - | - |
| 1 | 2 mM CaCl₂** | 0.037:1 | none | - | - |
| 1 | 5 mM CaCl₂** | 0.093:1 | none | - | - |
| 1 | 10 mM CaCl₂** | 0.185:1 | none | - | - |
| 1 | 40 mM CaCl₂** | 0.741:1 | none | - | - |
| 1 | 2 mM Ca(NO₃)₂** | 0.037:1 | none | - | - |
| 1 | 3 mM Ca(OH)₂ | 0.055:1 | none | - | - |
| 1 | 16 mM CaCl₂ | 0.296:1 | none | - | - |
| 3 | 1 mM CaCl₂ | 0.019:1 | none | none | none |
| 2,3 | 2 mM CaCl₂ | 0.037:1 | none | none | none |
| 3 | 3 mM CaCl₂ | 0.055:1 | none | none | none |
| 3 | 4 mM CaCl₂ | 0.074:1 | none | none | none |
| 3 | 7 mM CaCl₂ | 0.129:1 | none | none | none |
| 3 | 2 mM MgCl₂ | 0.037:1 | none | yes | - |
| 3 | 2 mM ZnCl₂ | 0.037:1 | yes | - | - |
| 3 | 2 mM NaCl | 0.037:1 | yes | - | - |
| 3 | 4 mM NaCl | 0.074:1 | yes | - | - |
| 2 | none | 0 | yes | - | - |

| | | | | | |
|---|---|---|---|---|---|
| * slight trace of aggregation, and aggregated on day 2. | | | | | |
| ** pH adjusted with NaOH | | | | | |
| - indicates that the test was not continued through this time period. | | | | | |

## Claims

1. A method for the prevention of aggregation of liposomes which are comprised of lipids and are dispersed in an aqueous medium which is external to the liposomes and which include a multivalent anion disposed on an outer surface of the liposome, the method characterized by having a divalent cation selected from the group consisting of calcium and magnesium present in the external medium.

2. The method of claim **1** in which the multivalent anion is selected from the group consisting of citrate, succinate, tartrate, oxalate, isocitrate, glutarate, fumarate, maleate, malonate, adipate, phthalate and sulfate.

3. The method of claim **1** or **2** in which the liposomes have a diameter of less that 200 nm.

4. The method of claim **1** or **2** in which the divalent cation is calcium.

5. The method of claim **3** in which the divalent cation is calcium.

6. The method of claim **1** or **2** in which the divalent cation is present in the aqueous medium in an amount of up to 1.5:1 mole ratio with respect to total lipid content of the liposomes.

7. The method of claim **3** in which the divalent cation is present in the aqueous medium in an amount of up to 1.5:1 mole ratio with respect to total lipid content of the liposomes.

8. The method of claim **4** in which the divalent cation is present in the aqueous medium in an amount of up to 1.5:1 mole ratio with respect to total lipid content of the liposomes.

9. The method of claim **5** in which the divalent cation is present in the aqueous medium in an amount of up to 1.5:1 mole ratio with respect to total lipid content of the liposomes.

10. The method of claim **1** or **2** in which the divalent cation is present in the aqueous medium in a mole ratio of from 0.002:1 to 0.9:1 with respect to total lipid content of the liposomes.

11. The method of claim **3** in which the divalent cation is present in the aqueous medium in a mole ratio of from 0.002:1 to 0.9:1 with respect to total lipid content of the liposomes.

12. The method of claim **4** in which the divalent cation is present in the aqueous medium in a mole ratio of from 0.002:1 to 0.9:1 with respect to total lipid content of the liposomes.

13. The method of claim **5** in which the divalent cation is present in the aqueous medium in a mole ratio of from 0.002:1 to 0.9:1 with respect to total lipid content of the liposomes.

14. A composition comprising liposomes which are comprised of lipids and which include a multivalent anion disposed on an outer surface thereof which are essentially free of aggregation in an external aqueous medium characterized by containing a divalent cation selected from the group consisting of calcium and magnesium.

15. The composition of claim **14** in which the multivalent anion is selected from the group consisting of citrate, succinate, tartrate, oxalate, iso-citrate, glutarate, fumarate, maleate, malonate, adipate, phthalate and sulfate.

16. The composition of claim **14** or **15** in which the liposomes have a diameter of less that 200 nm.

17. The composition of claim **14** or **15** in which the divalent cation is calcium.

18. The composition of claim **16** in which the divalent cation is calcium.

19. The composition of claim **14** or **15** in which the divalent cation is present in the aqueous medium in an amount of up to 1.5:1 mole ratio with respect to total lipid content of the liposome.

20. The composition of claim **16** in which the divalent cation is present in the aqueous medium in an amount of up to 1.5:1 mole ratio with respect to total lipid content of the liposomes.

21. The composition of claim **17** in which the divalent cation is present in the aqueous medium in an amount of up to 1.5:1 mole ratio with respect to total lipid content of the liposomes.

22. The composition of claim **18** in which the divalent cation is present in the aqueous medium in an amount of up to 1.5:1 mole ratio with respect to total lipid content of the liposomes.

23. The composition of claim **14** or **15** in which the divalent cation is present in the aqueous medium in a mole ratio of from 0.002:1 to 0.9:1 with respect to total lipid content of the liposomes.

24. The composition of claim **16** in which the divalent cation is present in the aqueous medium in a mole ratio of from 0.002:1 to 0.9:1 with respect to total lipid content of the liposomes.

25. The composition of claim **17** in which the divalent cation is present in the aqueous medium in a mole ratio of from 0.002:1 to 0.9:1 with respect to total lipid content of the liposomes.

26. The composition of claim **18** in which the divalent cation is present in the aqueous medium in a mole ratio of from 0.002:1 to 0.9:1 with respect to total lipid content of the liposomes.

## Patentansprüche

1. Verfahren zur Hemmung der Aggregation von Liposomen, welche aus Lipiden bestehen und in einem wässrigen Medium, das zu den Liposomen extern ist, dispergiert sind und welche ein auf einer äußeren Oberfläche des Liposoms angeordnetes mehrwertiges Anion einschließen, wobei das Verfahren dadurch gekennzeichnet ist, daß ein zweiwertiges Kation, ausgewählt aus der aus Calcium und Magnesium bestehenden Gruppe, in dem äußeren Medium vorhanden ist.

2. Verfahren nach Anspruch 1, in welchem das mehrwertige Anion aus der aus Citrat, Succinat, Tartrat, Oxalat, Isocitrat, Glutarat, Fumarat, Maleat, Malonat, Adipat, Phthalat und Sulfat bestehenden Gruppe ausgewählt ist.

3. Verfahren nach Anspruch 1 oder 2, in welchem die Liposome einen Durchmesser von weniger als 200 nm besitzen.

4. Verfahren nach Anspruch 1 oder 2, in welchem das zweiwertige Kation Calcium ist.

5. Verfahren nach Anspruch 3, in welchem das zweiwertige Kation Calcium ist.

6. Verfahren nach Anspruch 1 oder 2, in welchem das zweiwertige Kation in dem wässrigen Medium in einer Menge bis zum Molverhältnis 1,5 : 1, bezogen auf Gesamtlipidgehalt der Liposome, vorhanden ist.

7. Verfahren nach Anspruch 3, in welchem das zweiwertige Kation in dem wässrigen Medium in einer Menge bis zum Molverhältnis 1,5 : 1, bezogen auf Gesamtlipidgehalt der Liposome, vorhanden ist.

8. Verfahren nach Anspruch 4, in welchem das zweiwertige Kation in dem wässrigen Medium in einer Menge bis zum Molverhältnis 1,5 : 1, bezogen auf Gesamtlipidgehalt der Liposome, vorhanden ist.

9. Verfahren nach Anspruch 5, in welchem das zweiwertige Kation in dem wässrigen Medium in einer Menge bis zum Molverhältnis 1,5 : 1, bezogen auf Gesamtlipidgehalt der Liposome, vorhanden ist.

10. Verfahren nach Anspruch 1 oder 2, in welchem das zweiwertige Kation in dem wässrigen Medium in einem Molverhältnis von 0,002 : 1 bis 0,9 : 1, bezogen auf Gesamtlipidgehalt der Liposome, vorhanden ist.

11. Verfahren nach Anspruch 3, in welchem das Zweiwertige Kation in dem wässrigen Medium in einem Molverhältnis von 0,002 : 1 bis 0,9 : 1, bezogen auf Gesamtlipidgehalt der Liposome, vorhanden ist.

12. Verfahren nach Anspruch 4, in welchem das zweiwertige Kation in dem wässrigen Medium in einem Molverhältnis von 0,002 : 1 bis 0,9 : 1, bezogen auf Gesamtlipidgehalt der Liposome, vorhanden ist.

13. Verfahren nach Anspruch 5, in welchem das zweiwertige Kation in dem wässrigen Medium in einem Molverhältnis von 0,002 : 1 bis 0,9 : 1, bezogen auf Gesamtlipidgehalt der Liposome, vorhanden ist.

14. Zusammensetzung, umfassend Liposome, welche aus Lipiden bestehen und welche ein auf einer äußeren Oberfläche hiervon angeordnetes mehrwertiges Anion einschließen, welche im wesentlichen frei von Aggregation in einem äußeren wässrigen Medium sind, dadurch gekennzeichnet, daß sie ein zweiwertiges Nation, ausgewählt aus der aus Calcium und Magnesium bestehenden Gruppe enthält.

15. Zusammensetzung nach Anspruch 14, in welcher das mehrwertige Anion aus der aus Citrat, Succinat, Tartrat, Oxalat, Isocitrat, Glutarat, Fumarat, Maleat, Malonat, Adipat, Phthalat und Sulfat bestehenden Gruppe ausgewählt ist.

16. Zusammensetzung nach Anspruch 14 oder 15 in welcher die Liposome einen Durchmesser von weniger als 200 nm besitzen.

17. Zusammensetzung nach Anspruch 14 oder 15 in welcher das zweiwertige Kation Calcium ist.

18. Zusammensetzung nach Anspruch 16, in welcher das zweiwertige Kation Calcium ist.

19. Zusammensetzung nach Anspruch 14 oder 15, in welcher das zweiwertige Kation in dem wässrigen Medium in einer Menge bis zum Molverhältnis 1,5 : 1, bezogen auf Gesamtlipidgehalt des Liposoms, vorhanden ist.

20. Zusammensetzung nach Anspruch 16, in welcher das zweiwertige Kation in dem wässrigen Medium in einer Menge bis zum Molverhältnis 1,5 : 1, bezogen auf Gesamtlipidgehalt der Liposome, vorhanden ist.

21. Zusammensetzung nach Anspruch 17, in welcher das zweiwertige Kation in dem wässrigen Medium in einer Menge bis zum Molverhältnis 1,5 : 1, bezogen auf Gesamtlipidgehalt der Liposome, vorhanden ist.

22. Zusammensetzung nach Anspruch 18, in welcher das zweiwertige Kation in dem wässrigen Medium in einer Menge bis zum Molverhältnis 1,5 : 1, bezogen auf Gesamtlipidgehalt der Liposome, vorhanden ist.

23. Zusammensetzung nach Anspruch 14 oder 15, in welcher das zweiwertige Kation in dem wässrigen Medium in einem Molverhältnis von 0,002 : 1 bis 0,9 : 1, bezogen auf Gesamtlipidgehalt der Liposome, vorhanden ist.

24. Zusammensetzung nach Anspruch 16, in welcher das zweiwertige Kation in dem wässrigen Medium in einem Molverhältnis von 0,002 : 1 bis 0,9 : 1, bezogen auf Gesamtlipidgehalt der Liposome, vorhanden ist.

25. Zusammensetzung nach Anspruch 17, in welcher das zweiwertige Kation in dem wässrigen Medium in einem Molverhältnis von 0,002 : 1 bis 0,9 : 1, bezogen auf Gesamtlipidgehalt der Liposome, vorhanden ist.

26. Zusammensetzung nach Anspruch 18, in welcher das zweiwertige Kation in dem wässrigen Medium in einem Molverhältnis von 0,002 : 1 bis 0,9 : 1, bezogen auf Gesamtlipidgehalt der Liposome, vorhanden ist.

## Revendications

1. Procédé pour prévenir l'agrégation de liposomes constitués de lipides, dispersés dans un milieu aqueux qui est externe aux liposomes, et comprenant un anion plurivalent disposé sur une surface externe du liposome, le procédé étant caractérisé en ce qu'un cation divalent choisi parmi le calcium et le magnésium est présent dans le milieu externe.

2. Procédé selon la revendication 1, dans lequel l'anion plurivalent est choisi parmi le citrate, le succinate, le tartrate, l'oxalate, l'isocitrate, le glutarate, le fumarate, le maléate, le malonate, l'adipate, le phtalate et le sulfate.

3. Procédé selon la revendication 1 ou 2, dans lequel les liposomes ont un diamètre inférieur à 200 nm.

4. Procédé selon la revendication 1 ou 2, dans lequel le cation divalent est le calcium.

5. Procédé selon la revendication 3, dans lequel le cation divalent est le calcium.

6. Procédé selon la revendication 1 ou 2, dans lequel le cation divalent est présent dans le milieu aqueux dans une quantité allant jusqu'à un rapport stoechiométrique de 1,5/1 par rapport à la teneur totale en lipides des liposomes.

7. Procédé selon la revendication 3, dans lequel le cation divalent est présent dans le milieu aqueux dans une quantité allant jusqu'à un rapport stoechiométrique de 1,5/1 par rapport à la teneur totale en lipides des liposomes.

8. Procédé selon la revendication 4, dans lequel le cation divalent est présent dans le milieu aqueux dans une quantité allant jusqu'à un rapport stoechiométrique de 1,5/1 par rapport à la teneur totale en lipides des liposomes.

9. Procédé selon la revendication 5, dans lequel le cation divalent est présent dans le milieu aqueux dans une quantité allant jusqu'à un rapport stoechiométrique de 1,5/1 par rapport à la teneur totale en lipides des liposomes.

10. Procédé selon la revendication 1 ou 2, dans lequel le cation divalent est présent dans le milieu aqueux dans un rapport stoechiométrique de 0,002/1 à 0,9/1 par rapport à la teneur totale en lipides des liposomes.

11. Procédé selon la revendication 3, dans lequel le cation divalent est présent dans le milieu aqueux dans un rapport stoechiométrique de 0,002/1 à 0,9/1 par rapport à la teneur totale en lipides des liposomes.

12. Procédé selon la revendication 4, dans lequel le cation divalent est présent dans le milieu aqueux dans un rapport stoechiométrique de 0,002/1 à 0,9/1 par rapport à la teneur totale en lipides des liposomes.

13. Procédé selon la revendication 5, dans lequel le cation divalent est présent dans le milieu aqueux dans un rapport stoechiométrique de 0,002/1 à 0,9/1 par rapport à la teneur totale en lipides des liposomes.

14. Composition comprenant des liposomes constitués de lipides et contenant un anion plurivalent disposé sur leur surface externe, qui ne présentent sensiblement aucune agrégation dans un milieu aqueux externe, caractérisée en ce qu'elle contient un cation divalent choisi parmi le calcium et le magnésium.

15. Composition selon la revendication 14, dans laquelle l'anion plurivalent est choisi parmi le citrate, le succinate, le tartrate, l'oxalate, l'isocitrate, le glutarate, le fumarate, le maléate, le malonate, l'adipate, le phtalate et le sulfate.

16. Composition selon la revendication 14 ou 15, dans laquelle les liposomes ont un diamètre inférieur à 200 nm.

17. Composition selon la revendication 14 ou 15, dans laquelle le cation divalent est le calcium.

18. Composition selon la revendication 16, dans laquelle le cation divalent est le calcium.

19. Composition selon la revendication 14 ou 15, dans laquelle le cation divalent est présent dans le milieu aqueux dans une quantité allant jusqu'à un rapport stoechiométrique de 1,5/1 par rapport à la teneur totale en lipides des liposomes.

20. Composition selon la revendication 16, dans laquelle le cation divalent est présent dans le milieu aqueux dans une quantité allant jusqu'à un rapport stoechiométrique de 1,5/1 par rapport à la teneur totale en lipides des liposomes.

21. Composition selon la revendication 17, dans laquelle le cation divalent est présent dans le milieu aqueux dans une quantité allant jusqu'à un rapport stoechiométrique de 1,5/1 par rapport à la teneur totale en lipides des liposomes.

22. Composition selon la revendication 18, dans laquelle le cation divalent est présent dans le milieu aqueux dans une quantité allant jusqu'à un rapport stoechiométrique de 1,5/1 par rapport à la teneur totale en lipides des liposomes.

23. Composition selon la revendication 14 ou 15, dans laquelle le cation divalent est présent dans le milieu aqueux dans un rapport stoechiométrique de 0,002/1 à 0,9/1 par rapport à la teneur totale en lipides des liposomes.

24. Composition selon la revendication 16, dans laquelle le cation divalent est présent dans le milieu aqueux dans un rapport stoechiométrique de 0,002/1 à 0,9/1 par rapport à la teneur totale en lipides des liposomes.

25. Composition selon la revendication 17, dans laquelle le cation divalent est présent dans le milieu aqueux dans un rapport stoechiométrique de 0,002/1 à 0,9/1 par rapport à la teneur totale en lipides des liposomes.

26. Composition selon la revendication 18, dans laquelle le cation divalent est présent dans le milieu aqueux dans un rapport stoechiométrique de 0,002/1 à 0,9/1 par rapport à la teneur totale en lipides des liposomes.
